# EUROPEAN PATENT SPECIFICATION

(11) **EP 1 414 408 B1**
(45) Date of publication and mention of the grant of the patent: **29.03.2006**
(21) Application number: 02767353.2
(22) Date of filing: 09.08.2002
(51) Int. Cl.: A61K 9/00, A61K 47/26, A61K 31/443

(54) **COMPOSITION COMPRISING PAROXETINE AND A PHARMACEUTICALLY ACCEPTABLE GLYCYRRHIZINATE SALT**
ZUSAMMENSETZUNG ENTHALTEND PAROXETIN UND EIN PHARMAZEUTISCH VERTRÄGLICHES SALZ VON GLYCYRRHIZINSÄURE
COMPOSITION COMPRENANT DE LA PAROXETINE ET UN SEL DE GLYCYRRHIZINATE PHARMACEUTIQUEMENT ACCEPTABLE

(30) Priority: 09.08.2001 GB 0119470; 09.08.2001 GB 0119469; 09.08.2001 GB 0119467; 09.08.2001 GB 0119468
(43) Date of publication of application: 06.05.2004
(73) Proprietor: SMITHKLINE BEECHAM PLC, Brentford, Middlesex TW8 9GS (GB)
(72) Inventor: BARGES CAUSERET, Nathalie C. M., ZI du Terras -BP 2 53101 Mayenne, Cedex (FR); MARZOLINI, Nicola L. A. GlaxoSmithKline, Harlow, Essex CM19 5AW (GB); MENEAUD, Padma GlaxoSmithKline, Harlow Essex CM19 5AW (GB)
(74) Representative: Knight, Lucie Viktoria
(86) International application number: PCT/EP2002/008925
(87) International publication number: WO 2003/013470

(56) References cited:
- EP-A- 0 433 004
- WO-A-01/12161
- WO-A-02/074238
- US-A- 4 721 723
- US-A- 5 811 436
- J. FALBE, M. REGITZ: "Römpp Lexicon, 10. Auflage, Band 2 (Cm-G)" 1997 , THIEME , STUTTGART XP002220421 page 1576, column 1, paragraph GLYCYRRHIZIN

## Description

The present invention relates to a novel composition containing a pharmaceutically active compound, and to the use of the composition in therapy. In particular, this invention is concerned with a formulation of paroxetine that be taken orally by chewing or drinking without difficulties due to the bitter taste of paroxetine.

Pharmaceutical products with antidepressant and anti-Parkinson properties are described in US-A-3912743 and US-A-4007196. An especially important compound among those disclosed is paroxetine, the (-) *trans* isomer of 4-(4'-fluorophenyl)-3-(3',4'-methylenedioxy-phenoxymethyl)-piperidine. Paroxetine hydrochloride has been described in the literature as a crystalline hemihydrate (see EP-A-0223403 of Beecham Group) and as various crystalline anhydrate forms (see WO 96/24595 of SmithKline Beecham plc).

Paroxetine is used in therapy as the hydrochloride salt for the treatment and prophylaxis of *inter alia* depression, obsessive compulsive disorder (OCD) and panic, as compositions sold under the Registered Trade Marks SEROXAT and PAXIL.

From the International Search Report the following documents are known:
US 5811436 which proposes a complex between paroxetine and an ion-exchange resin to mask the bitterness of paroxetine;
WO 01/12161 which proposes sweetener combinations, including glycyrrhyzinates, for use with bitter tasting drugs;
Römpp Lexicon, 10 Auflage Band 2 (Cm-G), 1997 and EP 0433004 which disclose that glycyrrhyzinic acid and salts have been used sweeteners.

Because of its intensely bitter taste, paroxetine hydrochloride is generally administered to patients in swallow tablet form However, for some patients swallowing a whole tablet can be difficult, whereas swallowing a liquid medication or the residue of a chewed tablet is more easily carried out.

The present invention aims to satisfy the need for a formulation of paroxetine, especially hydrochloride, that can be present in a patient's mouth as a powder or granulate, for example as a chewed tablet, or as a liquid, for example by drinking a liquefied dispersible tablet or powder, without taste problems.

The present invention is based on the finding that glycyahyzinates rapidly associate with paroxetine in an aqueous medium greatly reducing the amount of paroxetine that is available as a bitter agent This has been enabled the inventors to satisfy the above mentioned need by providing a solid formulation of paroxetine in a therapeutically acceptable form and a glycynhyzinate salt in a composition which is dispersible in water or an aqueous medium, for example in a patient's mouth.

Accordingly, in one aspect of the invention there is provided a pharmaceutical composition which comprises a dry blend of paroxetine, or a salt or solvate thereof, a glycynhyzinate, and a dispersing agent which is dispersible in water or an aqueous medium.

The reference to paroxetine includes all forms of the compound in which paroxetine is available as a therapeutically effective agent. This includes paroxetine free base and pharmaceutically acceptable salts of paroxetine, especially paroxetine hydrochloride, particularly as the hemihydrate or one of the anhydrate forms, and paroxetine methanesulfonate (mesylate).

The glycyrrhyzinafe may be any pharmaceutically acceptable salt of glycyrrhyzinic acid, such as the disodium and dipotassium salts, or more preferably mono-ammonium glycyrrhyzinate.

Kinetic studies have shown that glycyrrhyzinates rapidly associate with paroxetine in an aqueous medium greatly reducing the amount of paroxetine that is available as a bitter agent. As a further advantage, the glycyrrhyzinate has itself an intense flavour of sweet liquorice providing further taste-masking effects. In fact, because of the intensity of the liquorice flavour, it is not necessary to provide the glycyrrhyzinate in an amount that will mask all the paroxetine present. Indeed further flavourings may be desirable to modify the taste of the formulation as a result of the presence of the glycyrrhyzinate.

The composition may be in powder form, especially with one or more conventional excipients, such as diluents, flavouring agents and sweeteners. Preferably a powder form is supplied as sealed sachets of the powder containing a unit dose of paroxetine.

Alternatively the powder may be loaded into capsule shells, which are broken to add the powder to an aqueous carrier, or as a chewable capsule.

The composition may also be provided as a shaped composition such as a tablet, in which case the composition typically includes one or more conventional excipients for tablet formation, such as mould lubricants and disintegrants. Tablets may be formulated to disintegrate in water, for dispersion as a suspension for swallowing by drinking, or as bite-dispersion or chewable tablets which are broken in the mouth by biting and dispersed in saliva for swallowing.

Suitable dispersing agents include polyvinyl pyrrolidone (such as Crospovidone XL, from ISP International Corp), calcium carbonate (such as Cal-Carb, from Whittaker, Clark & Daniels), and sodium starch glycolate (such as Explotab, from Edward Mendell Co Inc). These are incorporated into the formulation, singularly or in combination, to disperse the active ingredient in water after break-up of a tablet or addition of a powder to water, and to maintain the active ingredient in a dispersed form.

If desired, the composition may include further taste masking agents to modify the taste. Suitable agents includes potassium form polyacrylic acid ion exchange resins (such as Polacrilin K, from Rohm & Haas), β-cyclodextrin (such as Kleptose, from Roquette Inc), lecithin (such as Epikuron, from Lucas Meyer) and methacrylic acid copolymers (such as Eudragit L30D55, from Rohm & Haas). Alternatively the taste masking effect may be supplemented by an intense sweetener, such as those derived from fruit flavanoids.

In dispersible formulations, the relative quantities of the dispersing and taste masking agents may be adjusted to satisfy the desired balance of dispersability and taste masking. Also, the amount of dispersing agents relative to the other tabletting excipients may be adjusted to suit the desired requirements for the rate of break-up of the tablet in water. For example, dispersibility within 3 minutes may be achieved by using different disintegrating agents such as Polyplasdone (grade XL10 or 30) and Croscarmellose (Acdisol) in concentrations that can vary from 3 to 10 %. Addition of sodium laurylsulphate may also help to overcome the cohesiveness of for example, ammonium glycyrrhyzinate.

Typical excipients to make up the balance of the tablet formulation and to provide the requisite moldability and integrity of the tablet structure are conventional additives such as magnesium stearate and microcrystalline cellulose. The tablet may also contain sweeteners and flavourings to adjust the desired taste characteristics.

For use as a powder, the paroxetine, dispersing and taste masking agents may be blended as powders with other excipients such as solid diluents, flow control agents and desiccants, and then loaded into sachets or capsule shells by conventional means.

For chewable tablets, the composition of paroxetine and ammonium glycyrrhyzinate is dispersed in a conventional chewable base, for example containing lactose or mannitol.

It may also be advantageous to use the composition of paroxetine and glycyrrhyzinate in tablets and capsules intended for swallowing whole, in case a patient accidentally bites into the tablet and capsule, which in the absence of the glycyrrhyzinate could lead the patient to spit out the medication because of its bitter taste, and so disrupt the treatment regime.

When paroxetine is used in this invention as paroxetine hydrochloride, it is preferably in the form of the crystalline hemihydrate (see EP-A-0223403). However other crystalline forms may also be used such as crystalline anhydrates (see WO 96/24595), and other salts such as the maleate and acetate (see US-A-3912743 and US-A-4007196) and especially the mesylate.

Pharmaceutically acceptable salts of glycyrrhyzinic acid, such as the disodium and dipotassium salts, and mono-ammonium glycyrrhyzinate, are commercially available.

Therapeutic uses of the paroxetine containing compositions of this invention include treatment of alcoholism, anxiety, depression, obsessive compulsive disorder, panic disorder, chronic pain, obesity, senile dementia, migraine, bulimia, anorexia, social phobia, pre-menstrual syndrome (PMS), adolescent depression, trichotillomania, dysthymia, and substance abuse, referred to below as "the Disorders".

Accordingly, the present invention also provides:
the use of a dry blend of paroxetine and a glycyrrhyzinate for the manufacture of a medicament for the treatment or prophylaxis of one or more of the Disorders.

The present invention is illustrated by the following Examples.

### Example 1

| Paroxetine hydrochloride hemihydrate | |
|---|---|
| (equivalent to 20.0 mg free base) | 23.38 |
| Ammonium Glycyrrhyzinate | 32.00 |
| Polyplasdone XL-10 | 12.50 |
| Lactose | 117.50 |
| Microcrystalline Cellulose (Avicel PH200) | 58.70 |
| Magnesium stearate | 2.50 |
| Average weight | 246.58 mg |

### Example 2

| Paroxetine hydrochloride hemihydrate | |
|---|---|
| (equivalent to 20.0 mg free base) | 23.38 |
| Ammonium Glycyrrhyzinate | 32.00 |
| Polyplasdone XL-10 | 12.50 |
| Lactose | 154.20 |
| Aspartame | 12.00 |
| Magnesium stearate | 2.50 |
| Average weigh | 250.58 mg |

### Example 3

| Paroxetine hydrochloride hemihydrate | |
|---|---|
| (equivalent to 20.0 mg free base) | 23.38 |
| Ammonium Glycyrrhyzinate | 32.00 |
| Polyplasdone XL-10 | 12.50 |
| Lactose Fast-Flow | 117.50 |
| Pearlitol SD-200 | 36.70 |
| Flavour 597924P-1051 | 14.00 |
| Aspartam | 12.00 |
| Magnesium stearate | 2.50 |
| Average weight | 250.58 |

### Example 4

| Paroxetine hydrochloride hemihydrate | |
|---|---|
| (equivalent to 20.0 mg free base) | 23.38 |
| Ammonium Glycyrrhyzinate | 10.00 |
| Special Flavour * | 14.00 |
| Polyplasdone XL-10 | 12.50 |
| Atomized lactose | 117.50 |
| Microcrystalline cellulose PH200 | 58.70 |
| Aspartame | 12.00 |
| Magnesium stearate | 2.50 |
| Average weight | 251.28 mg |

| | |
|---|---|
| * here a relatively neutral aroma which can be replaced as desired, for example by cherry, orange, tangerine or cassis flavours. | |

### Example 5

| Paroxetine hydrochloride hemihydrate | |
|---|---|
| (equivalent to 20.0 mg free base) | 23.38 |
| Ammonium Glycyrrhyzinate | 10.00 |
| Cherry 501150 APO551(Firmenich) | 16.00 |
| Polyplasdone XL-10 | 12.50 |
| Atomized lactose | 117.50 |
| Microcrystalline cellulose pH200 | 58.70 |
| Aspartame | 12.00 |
| Magnesium stearate | 2.50 |
| Total weight | 252.58 |

### Example 6

| | |
|---|---|
| Paroxetine hydrochloride | 22.80 |
| Ammonium glycyrrhyzinate | 14.00 |
| Crospovidone | 25.00 |
| Pearlitol SD200 | 73.00 |
| Microcrystalline cellulose pH200 | 100.00 |
| Aspartame | 12.00 |
| Magnesium stearate | 2.50 |
| Total weight | |

### Example 7

| | |
|---|---|
| Paroxetine hydrochloride | 22.80 |
| Ammonium glycyrrhyzinate | 14.00 |
| Crospovidone | 25.00 |
| Pearlitol SD200 | 63.00 |
| Microcrystalline cellulose pH200 | 82.00 |
| Cherry flavour | 18.00 |
| Aspartame | 12.00 |
| Magnesium stearate | 2.50 |
| Total weight | |

### Example 8

| | |
|---|---|
| Paroxetine hydrochloride | 22.80 |
| Ammonium glycyrrhyzinate | 2.50 |
| Amberlite IRP88 | 42.10 |
| Crospovidone | 25.00 |
| Pearlitol SD200 | 14.00 |
| Microcrystalline cellulose PH200 | 62.00 |
| Microcrystalline cellulose PH102 | 69.60 |
| Syloid 244 | 0.50 |
| Aspartame | 20.00 |
| Magnesium stearate | 2.50 |
| Total weight | |

## Claims

1. A pharmaceutical composition which is a dry blend of :
paroxetine free base, or a pharmaceutically acceptable salt or solvate of paroxetine;
a pharmaceutically acceptable glycyrrhyzinate salt; and
a dispersing agent which is dispersible in water or an aqueous medium.

2. A composition according to claim 1 which is in powder form.

3. A composition according to claim 1 which is a shaped composition.

4. A composition according to claim 1, 2 or 3 in which the dispersing agent is selected from polyvinyl pyrrolidone, calcium carbonate and sodium starch glycolate.

5. A composition according to any one of claims 1 to 4 including one or more taste masking agents, sweeteners or flavourings.

6. A composition according to any one of the preceding claims in which the paroxetine salt is paroxetine hydrochloride.

7. A composition according to any one of the preceding claims in which the glycyrrhyzinate salt is ammonium glycyrrhyzinate.

8. Use of a composition according to any one of the preceding claims for the preparation of a medicament for the treatment or prophylaxis of one or more disorders selected from alcoholism, anxiety, depression, obsessive compulsive disorder, panic disorder, chronic pain, obesity, senile dementia, migraine, bulimia, anorexia, social phobia, pre-menstrual syndrome, adolescent depression, trichotillomania, dysthymia and substance abuse.

## Patentansprüche

1. Pharmazeutische Zusammensetzung, welche eine Trockenmischung ist aus:
Paroxetin freie Base oder einem pharmazeutisch verträglichen Salz oder Solvat von Paroxetin;
einem pharmazeutisch verträglichen Glycyrrhyzinatsalz; und
einem Dispergiermittel, welches in Wasser oder einem wässrigen Medium dispergierbar ist.

2. Zusammensetzung gemäß Anspruch 1, welche in Pulverform vorliegt.

3. Zusammensetzung gemäß Anspruch 1, welche eine geformte Zusammensetzung ist.

4. Zusammensetzung gemäß Anspruch 1, 2 oder 3, in welcher das Dispergiermittel ausgewählt ist aus Polyvinylpyrrolidon, Calciumcarbonat und Natriumstärkeglycolat.

5. Zusammensetzung gemäß einem der Ansprüche 1 bis 4, einschließlich eines oder mehrerer Mittel zur Geschmacksüberdeckung, Süßungsmittel oder Aromastoffe.

6. Zusammensetzung gemäß einem der vorstehenden Ansprüche, in welcher das Paroxetinsalz Paroxetinhydrochlorid ist.

7. Zusammensetzung gemäß einem der vorstehenden Ansprüche, in welcher das Glycyrrhyzinatsalz Ammoniumglycyrrhyzinat ist.

8. Verwendung einer Zusammensetzung gemäß einem der vorstehenden Ansprüche zur Herstellung eines Medikaments zur Behandlung oder Prophylaxe einer oder mehrerer Störungen, ausgewählt aus Alkoholismus, Angst, Depression, Zwangsstörung, Panikstörung, chronischem Schmerz, Fettsucht, seniler Demenz, Migräne, Bulemie, Anorexie, sozialer Phobie, prämenstruellem Syndrom, Depression in der Adoleszenz, Haarrupfsucht, Dysthymie und Drogenmissbrauch.

## Revendications

1. Composition pharmaceutique qui est un mélange sec :
de paroxétine sous forme de base libre, ou d'un sel ou produit de solvatation pharmaceutiquement acceptable de paroxétine ;
d'un sel glycyrrhizinate pharmaceutiquement acceptable ; et
d'un agent dispersant qui est dispersible dans l'eau ou un milieu aqueux.

2. Composition suivant la revendication 1, qui est sous forme de poudre.

3. Composition suivant la revendication 1, qui est une composition façonnée.

4. Composition suivant la revendication 1, 2 ou 3, dans laquelle l'agent dispersant est choisi entre la polyvinylpyrrolidone, le carbonate de calcium et le glycolate d'amidon sodique.

5. Composition suivant l'une quelconque des revendications 1 à 4, comprenant un ou plusieurs agents masquant le goût, agents édulcorants ou agents aromatisants.

6. Composition suivant l'une quelconque des revendications précédentes, dans laquelle le sel de paroxétine est le chlorhydrate de paroxétine.

7. Composition suivant l'une quelconque des revendications précédentes, dans laquelle le sel glycyrrhizinate est le glycyrrhizinate d'ammonium.

8. Utilisation d'une composition suivant l'une quelconque des revendications précédentes pour la préparation d'un médicament destiné au traitement ou à la prophylaxie d'une ou plusieurs affections choisies entre l'alcoolisme, l'anxiété, la dépression, le syndrome obsessionnel compulsif, le trouble panique, la douleur chronique, l'obésité, la démence sénile, la migraine, la boulimie, l'anorexie, la névrose phobique, le syndrome prémenstruel, la dépression de l'adolescence, la trichotillomanie, la dysthymie et la pharmacodépendance.
